# EUROPÄISCHE PATENTSCHRIFT

(11) **EP 0 827 497 B1**
(45) Veröffentlichungstag und Bekanntmachung des Hinweises auf die Patenterteilung: **15.11.2000**
(21) Anmeldenummer: 96914117.5
(22) Anmeldetag: 25.04.1996
(51) Int. Cl.: C07D 239/54, C07D 409/12, A01N 43/54

(54) **SUBSTITUIERTE AMINOPHENYLURACILE ALS HERBIZIDE UND INSEKTIZIDE**
SUBSTITUTED AMINOPHENYLURACILS AS HERBICIDES AND INSECTICIDES
AMINOPHENYLURACILES SUBSTITUES UTILISES COMME HERBICIDES ET COMME INSECTICIDES

(30) Priorität: 08.05.1995 DE 19516785
(43) Veröffentlichungstag der Anmeldung: 11.03.1998
(73) Patentinhaber: BAYER AG, 51368 Leverkusen (DE)
(72) Erfinder: ANDREE, Roland, D-40764 Langenfeld (DE); DREWES, Mark, Wilhelm, D-40764 Langenfeld (DE); DOLLINGER, Markus, D-51381 Leverkusen (DE); SANTEL, Hans-Joachim, D-51371 Leverkusen (DE); ERDELEN, Christoph, D-42799 Leichlingen (DE)
(86) Internationale Anmeldenummer: EP9601722
(87) Internationale Veröffentlichungsnummer: WO9635679

(56) Entgegenhaltungen:
- EP-A- 0 438 209
- EP-A- 0 563 384
- DE-A- 4 437 295
- US-A- 5 084 084

## Beschreibung

Die Erfindung betrifft neue substituierte Aminophenyluracile, ein Verfahren zu ihrer Herstellung und ihre Verwendung als Herbizide und Insektizide.

Es ist bekannt, dass bestimmte substituierte Aminophenyluracile herbizide Eigenschaften aufweisen (vgl. EP 308382/US 5127935/US5154755, EP 563384, DE 4412079). EP-A-438 209 zeigt Uracil-Drivate, die pestizide Eigenschaften aufweisen. Diese Verbindung haben jedoch bisher weder als Herbizide noch als Insektizide nennenswerte Bedeutung erlangt.

Es wurden nun die neuen substituierten Aminophenyluracile der allgemeinen Formel (I) gefunden 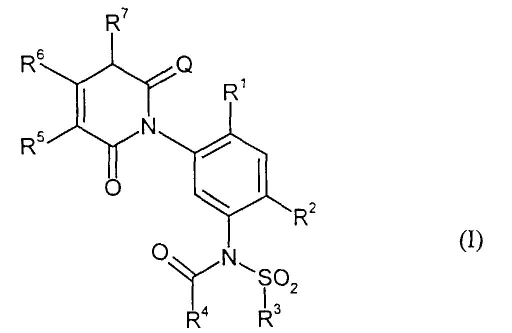 in welcher
- Q: für Sauerstoff oder Schwefel steht,
- R¹: für Wasserstoff, Cyano oder Halogen steht,
- R²: für Cyano, Thiocarbamoyl oder für gegebenenfalls substituiertes Alkyl steht,
- R³: für jeweils gegebenenfalls substituiertes Alkyl, Cycloalkyl, Aryl, Arylalkyl oder Heteroaryl steht,
- R⁴: für jeweils gegebenenfalls substituiertes Cycloalkyl, Aryl oder Heteroaryl steht,
- R⁵: für Wasserstoff, Halogen oder für jeweils gegebenenfalls substituiertes Alkyl oder Alkoxy steht,
- R⁶: für gegebenenfalls substituiertes Alkyl steht und
- R⁷: für Wasserstoff, Hydroxy, Amino oder für jeweils gegebenenfalls substituiertes Alkyl, Alkoxy, Alkenyl oder Alkinyl steht ausgenommen
Verbindungen der folgenden Formeln: 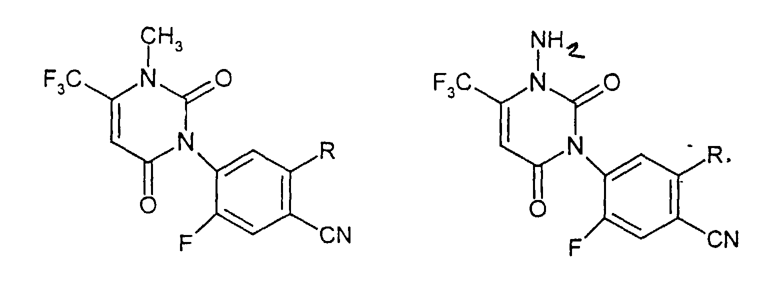 wobei
- R: für N(SO₂Me)CO(cyclopropyl),
N(SO₂Me)CO Ph,
N(SO₂Et)CO(cyclopropyl) oder
N(SO₂Et)CO Ph steht.

Man erhält die neuen substituierten Aminophenyluracile der allgemeinen Formel (I), wenn man entsprechende Sulfonylaminophenyl-uracile der allgemeinen Formel (II) 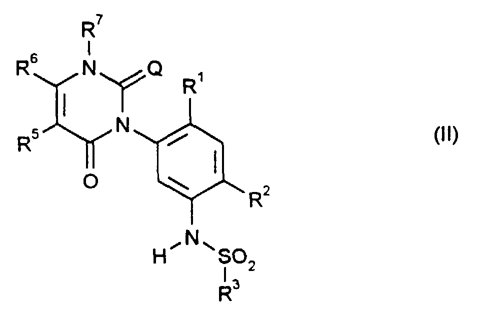 in welcher
Q, R¹, R², R³, R⁵, R⁶ und R⁷ die oben angegebenen Bedeutungen haben,
mit Säurederivaten der allgemeinen Formel (III)

R⁴-CO-X (III)

in welcher
- R⁴: die oben angegebene Bedeutung hat und
- X: für Halogen oder die Gruppierung -O-CO-R⁴ steht,
gegebenenfalls in Gegenwart eines Reaktionshilfsmittels und gegebenenfalls in Gegenwart eines Verdünnungsmittels umsetzt.

Die neuen substituierten Aminophenyluracile der allgemeinen Formel (I) zeichnen sich durch starke herbizide und insektizide Wirksamkeit aus.

In den Definitionen sind die gesättigten oder ungesättigten Kohlenwasserstoffketten, wie Alkyl, Alkenyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- Q: für Sauerstoff oder Schwefel steht,
- R¹: für Wasserstoff, Cyano, Fluor oder Chlor steht,
- R²: für Cyano, Thiocarbamoyl, oder für gegebenenfalls durch Fluor und/oder Chlor substituiertes Alkyl mit 1 bis 4 Kohlenstoffatomen steht,
- R³: für gegebenenfalls durch Cyano, Fluor, Chlor, Brom, C₁-C₄-Alkoxy oder Cₗ-C₄-Alkylthio substituiertes Alkyl mit 1 bis 6 Kohlenstoffatomen steht,
- R³: weiterhin für gegebenenfalls durch Cyano, Fluor, Chlor, Brom oder C₁-C₄-Alkyl substituiertes Cycloalkyl mit 3 bis 8 Kohlenstoffatomen steht,
- R³: weiterhin für Phenyl, Naphthyl, Benzyl, Phenylethyl, Thienyl, Pyrazolyl, Pyridinyl oder Chinolinyl steht,
wobei als Substituenten jeweils gegebenenfalls in Frage kommen:
Fluor, Chlor, Brom, Cyano, Nitro, Carboxy, Carbamoyl, Thiocarbamoyl, C₁-C₄-Alkyl, C₁-C₄-Alkoxy, C₁-C₄-Alkylthio, Dimethylaminosulfonyl, Diethylaminosulfonyl, jeweils gegebenenfalls durch Fluor und/oder Chlor substituiertes C₁-C₄-Alkylsulfinyl oder C₁-C₄-Alkylsulfonyl, jeweils gegebenenfalls durch Fluor, Chlor, Brom, Cyano, Methoxy oder Ethoxy substituiertes C₁-C₄-Alkoxycarbonyl oder jeweils gegebenenfalls durch Fluor, Chlor, Brom, Cyano, Methyl, Methoxy, Trifluormethyl und/oder Trifluormethoxy substituiertes Phenyl, Phenyloxy oder Phenylthio;
- R⁴: für gegebenenfalls durch Cyano, Fluor, Chlor, Brom oder C₁-C₄-Alkyl substituiertes Cycloalkyl mit 3 bis 8 Kohlenstoffatomen steht,
- R⁴: weiterhin für jeweils gegebenenfalls substituiertes Phenyl, Naphthyl, Furyl, Thienyl, Oxazolyl, Isoxazolyl, Pyrazolyl, Pyridinyl, Pyrimidinyl oder Chinolinyl steht, wobei als Substituenten jeweils gegebenenfalls in Frage kommen:
Fluor, Chlor, Brom, Cyano, Nitro, Carboxy, Carbamoyl, Thiocarbamoyl, Dimethylamino, Dimethylaminosulfonyl, Diethylaminosulfonyl, jeweils gegebenenfalls durch Fluor und/oder Chlor substituiertes C₁-C₄-Alkyl, C₁-C₄-Alkoxy, C₁-C₄-Alkylthio, C₁-C₄-Alkylsulfinyl oder C₁-C₄-Alkylsulfonyl oder gegebenenfalls durch Fluor, Chlor, Brom, Cyano, Methoxy oder Ethoxy substituiertes C₁-C₄-Alkoxycarbonyl;
- R⁵: für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
- R⁶: für gegebenenfalls durch Fluor und/oder Chlor substituiertes Alkyl mit 1 bis 4 Kohlenstoffatomen steht und
- R⁷: für Wasserstoff, Hydroxy, Amino oder für jeweils gegebenenfalls durch Fluor, Chlor oder C₁-C₄-Alkoxy substituiertes Alkyl, Alkoxy, Alkenyl oder Alkinyl mit jeweils bis zu 6 Kohlenstoffatomen steht.

Die Erfindung betrifft insbesondere Verbindungen der Formel (I), in welcher
- Q: für Sauerstoff oder Schwefel steht,
- R¹: für Wasserstoff, Cyano, Fluor oder Chlor steht,
- R²: für Cyano, Thiocarbamoyl, Methyl, Ethyl, n- oder i-Propyl, n-, i-, s- oder t-Butyl oder Trifluormethyl steht,
- R³: für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
- R³: weiterhin für jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl steht,
- R³: weiterhin für jeweils gegebenenfalls substituiertes Phenyl, Naphthyl, Benzyl, Phenylethyl, Thienyl, Pyrazolyl, Pyridinyl oder Chinolinyl steht, wobei als Substituenten infrage kommen: Fluor, Chlor, Brom, Cyano, Nitro, Carboxy, Carbamoyl, Thiocarbamoyl, Methyl, Ethyl, n- oder i-Propyl, Trifluormethyl, Methoxy, Ethoxy, n- oder i-Propoxy, Difluormethoxy, Trifluormethoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Dimethylaminosulfonyl, Diethylaminosulfonyl, Methoxycarbonyl, Ethoxycarbonyl, n- oder i-Propoxycarbonyl,
- R⁴: für gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl steht,
- R⁴: weiterhin für jeweils gegebenenfalls substituiertes Phenyl, Naphthyl, Furyl, Thienyl, Oxazolyl, Isoxazolyl, Pyrazolyl, Pyridinyl oder Pyrimidinyl steht, wobei als Substituenten jeweils gegebenenfalls in Frage kommen: Fluor, Chlor, Brom, Cyano, Nitro, Carboxy, Carbamoyl, Thiocarbamoyl, Dimethylamino, Dimethylaminosulfonyl oder Diethylaminosulfonyl, jeweils gegebenenfalls durch Fluor und/oder Chlor substituiertes Methyl, Ethyl, n-oder i-Propyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, jeweils gegebenenfalls durch Fluor, Chlor, Brom, Cyano, Methoxy oder Ethoxy substituiertes Methoxy-carbonyl, Ethoxycarbonyl, n- oder i-Propoxycarbonyl, jeweils gegebenenfalls durch Fluor, Chlor, Brom, Cyano, Methyl, Methoxy, Trifluormethyl und/oder Trifluormethoxy substituiertes Phenyl, Phenyloxy oder Phenylthio,
- R⁵: für Wasserstoff, Fluor, Chlor, Brom oder für jeweils gegebenenfalls durch Fluor und/oder Chlor substituiertes Methyl, Ethyl, n- oder i-Propyl steht,
- R⁶: für gegebenenfalls durch Fluor und/oder Chlor substituiertes Methyl, Ethyl, n- oder i-Propyl steht und
- R⁷: für Wasserstoff, Amino oder für jeweils gegebenenfalls durch Fluor und/oder Chlor substituiertes Methyl, Ethyl, n- oder i-Propyl, n-, i- oder s-Butyl, Methoxy, Ethoxy, n- oder i-Propoxy, n-, i- oder s-Butoxy, Propenyl, Butenyl, Propinyl oder Butinyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangsstoffe bzw. Zwischenprodukte. Diese Restedefinitionen können untereinander, also auch zwischen den angegebenen bevorzugten Bereichen beliebig kombiniert werden.

Verwendet man beispielsweise 1-(4-Chlor-2-fluor-5-methylsulfonylamino-phenyl)-3,6-dihydro-2,6-dioxo-3,4-dimethyl-1(2H)-pyrimidin und 2-Fluor-benzoylchlorid als Ausgangsstoffe, so kann der Reaktionsablauf beim erfindungsgemäßen Verfahren durch das folgende Formelschema skizziert werden:

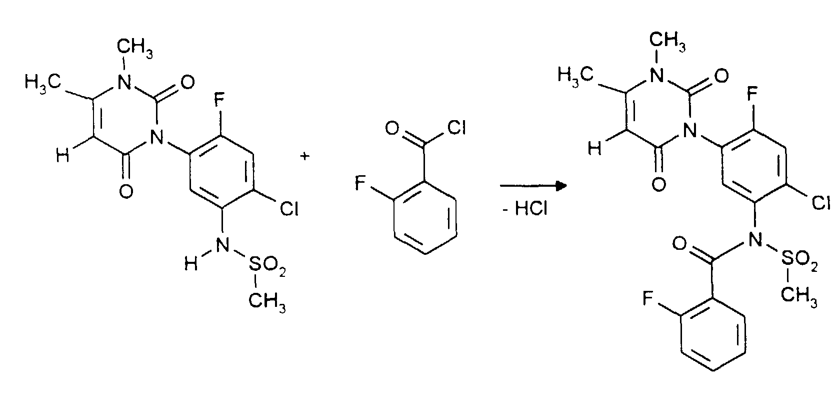

Die beim erfindungsgemäßen Verfahren zur Herstellung der Verbindungen der Formel (I) als Ausgangsstoffe zu verwendenden Sulfonylaminophenyl-uracile sind durch die Formel (II) allgemein definiert. In der Formel (II) haben Q, R¹, R², R³, R⁵, R⁶ und R⁷ vorzugsweise bzw. insbesondere diejenige Bedeutung, die bereits oben in der Beschreibung der erfindungsgemäß herzustellenden Verbindungen der Formel (I) vorzugsweise bzw. als insbesondere bevorzugt für Q, R¹, R², R³, R⁵, R⁶ und R⁷ angegeben wurde.

Die Ausgangsstoffe der Formel (II) sind bekannt und/oder können nach an sich bekannten Verfahren hergestellt werden (vgl. EP 408382/US 5084084/US 5127935/US5154755, EP 563384, DE 4412079).

Die beim erfindungsgemäßen Verfahren zur Herstellung der Verbindungen der Formel (I) weiter als Ausgangsstoffe zu verwendenden Säurehalogenide sind durch die Formel (III) allgemein definiert. In der Formel (III) hat R⁴ vorzugsweise bzw. insbesondere diejenige Bedeutung, die bereits oben bei der Beschreibung der erfindungsgemäß herzustellenden Verbindungen der Formel (I) vorzugsweise bzw. als insbesondere bevorzugt für R⁴ angegeben wurde; X steht vorzugsweise für Fluor, Chlor oder Brom, insbesondere für Chlor.

Die Ausgangsstoffe der Formel (III) sind bekannte Synthesechemikalien.

Das erfindungsgemäße Verfahren zur Herstellung der Verbindungen der Formel (I) wird vorzugsweise in Gegenwart eines geeigneten Reaktionshilfsmittels durchgeführt. Als Reaktionshilfsmittel kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calciumhydrid, Lithium-, Natrium-, Kalium- oder Calcium-hydroxid, Natrium- oder Kalium- -methanolat, -ethanolat, n- oder i-propanolat, n-, i-, s- oder t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Das erfindungsgemäße Verfahren zur Herstellung der Verbindungen der Formel (I) wird vorzugsweise in Gegenwart eines Verdünnungsmittels durchgeführt. Als Verdünnungsmittel kommen im allgemeinen die üblichen organischen Lösungsmittel in Betracht. Hierzu gehören vorzugsweise aliphatische, alicyclische und aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan (Methylenchlorid), Trichlormethan (Chloroform) oder Tetrachlormethan, Dialkylether, wie beispielsweise Diethylether, Diisopropylether, Methyl-t-butylether, Ethyl-t-butylether, Methyl-t-pentylether (MTBE), Ethyl-t-pentylether, Tetrahydrofuran (THF), 1,4-Dioxan, Ethylenglycol-dimethylether oder -diethylether, Diethylenglycoldimethylether oder -diethylether; Dialkylketone, wie beispielsweise Aceton, Butanon (Methylethylketon), Methyl-i-propylketon oder Methyl-i-butylketon, Nitrile, wie beispielsweise Acetonitril, Propionitril, Butyronitril oder Benzonitril; Amide, wie beispielsweise N,N-Dimethyl-formamid (DMF), N,N-Dimethyl-acetamid, N-Methyl-formanilid, N-Methyl-pyrrolidon oder Hexamethyl-phosphorsäuretriamid; Ester, wie beispielsweise Essigsäure-methylester, -ethylester, -n- oder -i-propylester, -n-, -i- oder -s-butylester; Sulfoxide, wie beispielsweise Dimethylsulfoxid; Alkanole, wie beispielsweise Methanol, Ethanol, n- oder i-Propanol, n-, i-, s- oder t-Butanol, Ethylenglycol-monomethylether oder -monoethylether, Diethylenglycol-monomethylether oder -monoethylether; deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen -10°C und +150°C, vorzugsweise bei Temperaturen zwischen 0°C und 100°C.

Das erfindungsgemäße Verfahren wird im allgemeinen bei atmosphärischem Druck ("Normaldruck") durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen der Formel (I) eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Macrosiphum avenae, Myzus spp., Pemphigus spp., Phorodon humuli, Phylloxera vastatrix, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Bruchidius obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.. Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..
Zu den pflanzenparasitären Nematoden gehören z.B. Pratylenchus spp., Radopholus spp., Ditylenchus spp., Tylenchulus spp., Heterodera spp., Globodera spp., Meloidogyne spp., Aphelenchoides spp., Longidorus spp., Xiphinema spp., Trichodorus spp., Tylenchus spp., Helicotylenchus spp., Rotylenchus spp., Tylenchulus spp..

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfs-lösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyanin-farbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuronmethyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

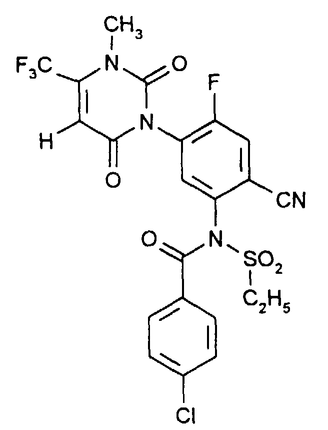

0,90 g (5 mMol) 4-Chlor-benzoylchlorid werden bei ca. 20°C zu einer Mischung aus 2,1 g (5 mMol) 1-(4-Cyano-5-ethylsulfonylamino-2-fluor-phenyl)-3,6-dihydro-2,6-dioxo-3-methyl-4-trifluormethyl-1(2H)-pyrimidin, 0,60 g (6 mMol) Triethylamin und 50 ml Acetonitril gegeben und die Reaktionsmischung wird 2 Stunden bei 20°C gerührt. Dann wird im Wasserstrahlvakuum eingeengt, der Rückstand in Chloroform aufgenommen, mit 2N-Salzsäure gewaschen, mit Natriumsulfat getrocknet und filtriert. Das Filtrat wird im Wasserstrahlvakuum eingeengt, der Rückstand mit Diethylether digeriert und das hierbei kristallin anfallende Produkt durch Absaugen isoliert.

Man erhält 1,5 g (54% der Theorie) 1-[4-Cyano-5-(N-ethylsulfonyl-N-(4-chlorbenzoyl)amino)-2-fluor-phenyl]-3,6-dihydro-2,6-dioxo-3-methyl-4-trifluormethyl-1(2H)-pyrimidin vom Schmelzpunkt 138°C.

### Beispiel 2

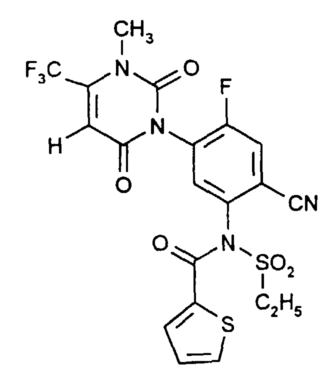

1,8 g (12 mMol) Thiophen-2-carbonsäurechlorid werden bei ca. 20°C zu einer Mischung aus 2,1 g (5 mMol) 1-(4-Cyano-5-ethylsulfonylamino-2-fluor-phenyl)-3,6-dihydro-2,6-dioxo-3-methyl-4-trifluormethyl-1(2H)-pyrimidin, 1,5 ml Pyridin und 50 ml Methylenchlorid gegeben und die Reaktionsmischung wird eine Woche bei 20°C gerührt. Dann wird die Lösung mit 1N-Salzsäure gewaschen, mit Natriumsulfat getrocknet und filtriert. Das Filtrat wird im Wasserstrahlvakuum eingeengt, der Rückstand mit Diethylether digeriert und das hierbei kristallin angefallene Produkt durch Absaugen isoliert.

Man erhält 2,1 g (84% der Theorie) 1-[4-Cyano-5-(N-ethylsulfonyl-N-(thien-2-yl-carbonyl)amino)-2-fluor-phenyl]-3,6-dihydro-2,6-dioxo-3-methyl-4-trifluormethyl-1(2H)-pyrimidin vom Schmelzpunkt 192°C.

Analog Beispiel 1 und 2 sowie entsprechend der allgemeinen Beschreibung des erfindungsgemäßen Herstellungsverfahrens können beispielsweise auch die in der nachstehenden Tabelle 1 aufgeführten Verbindungen der Formel (I) hergestellt werden.

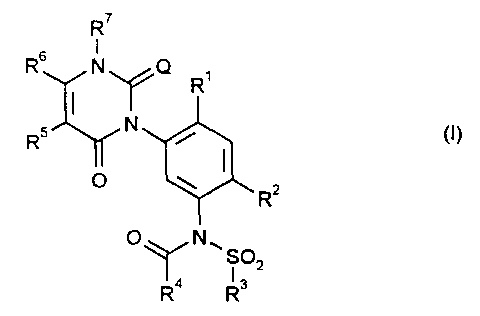

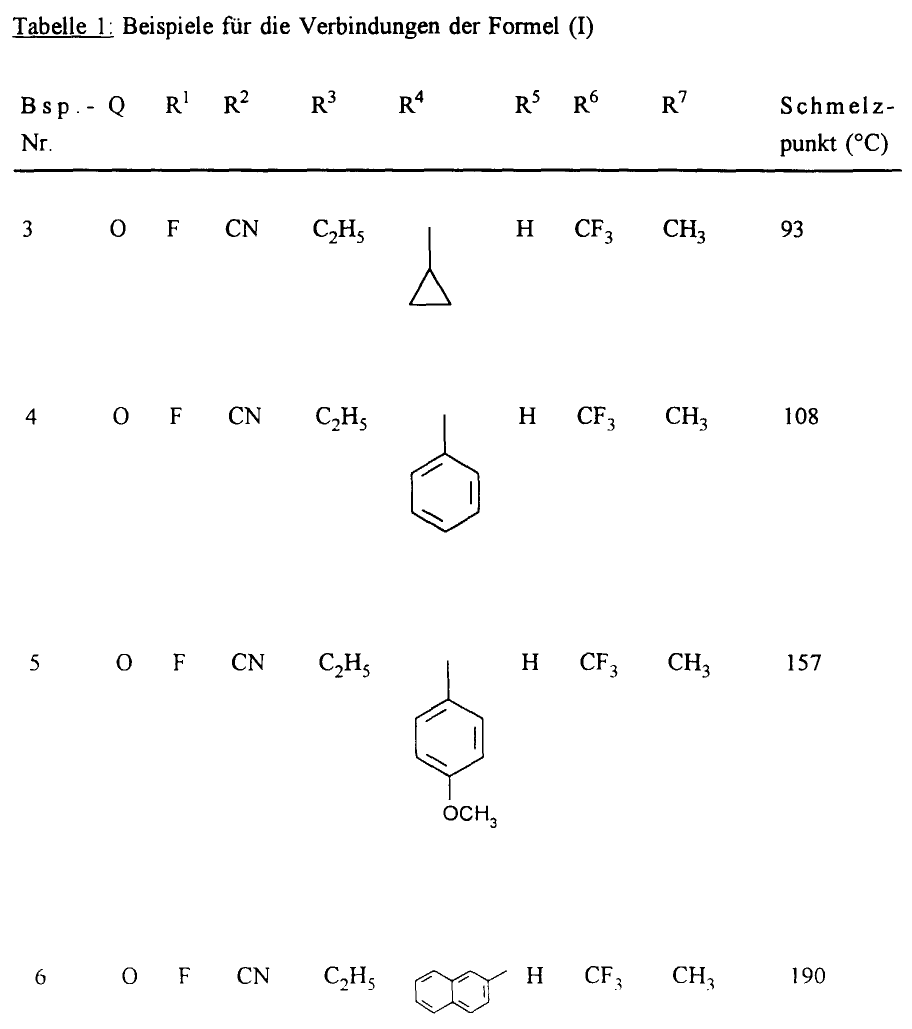

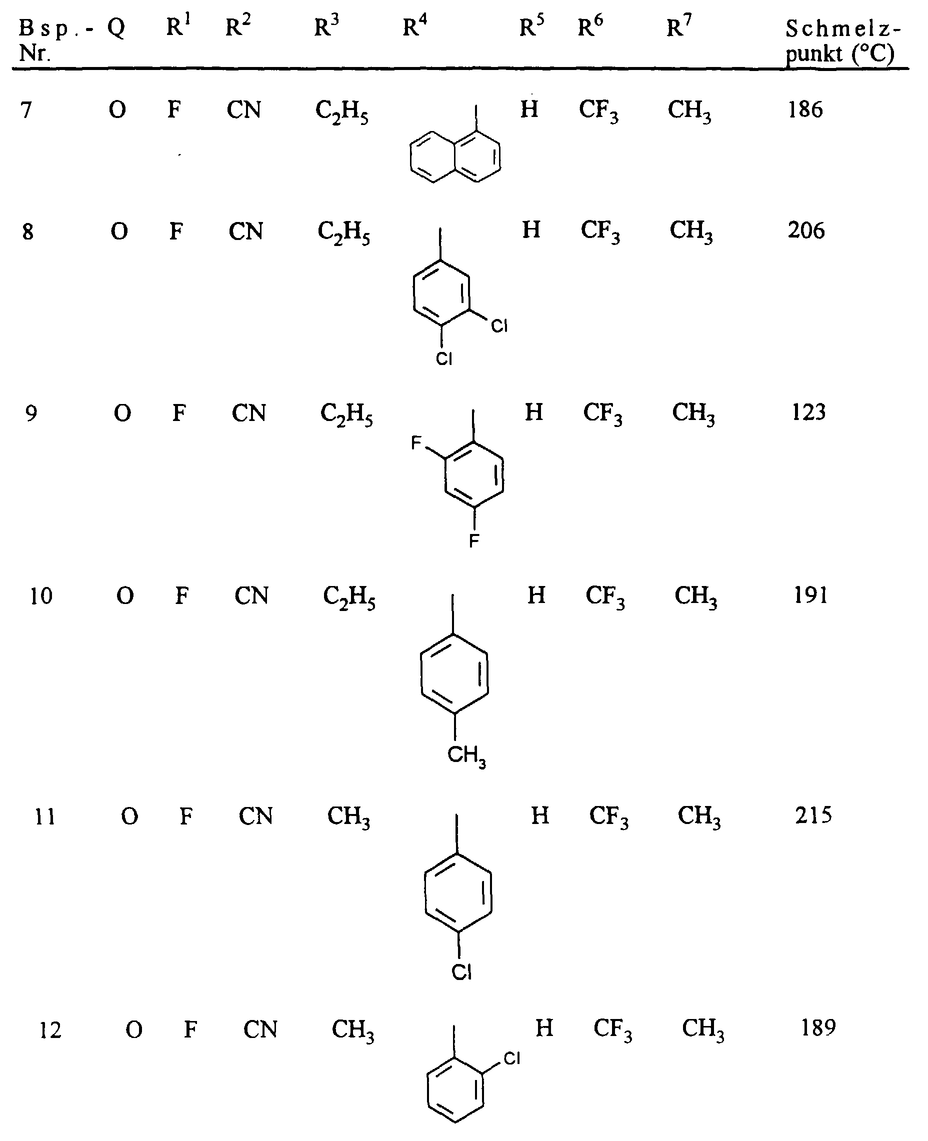

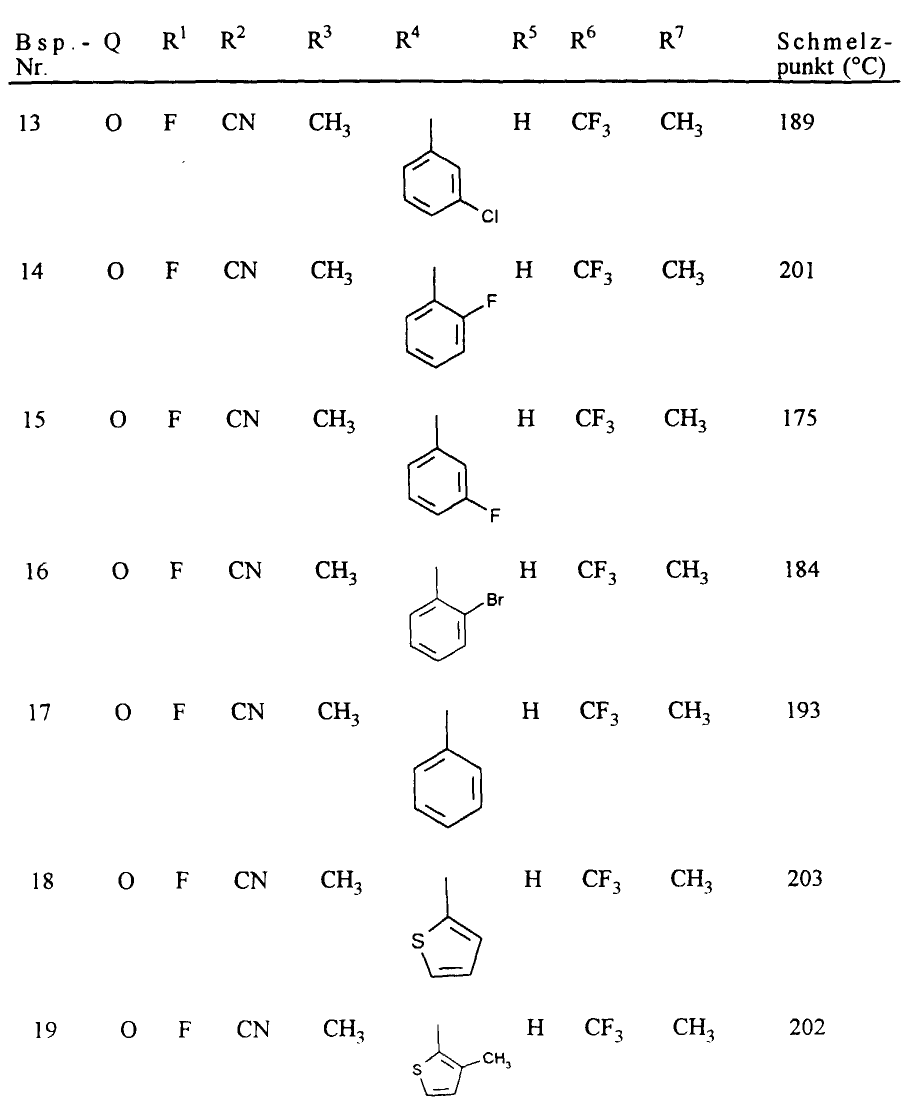

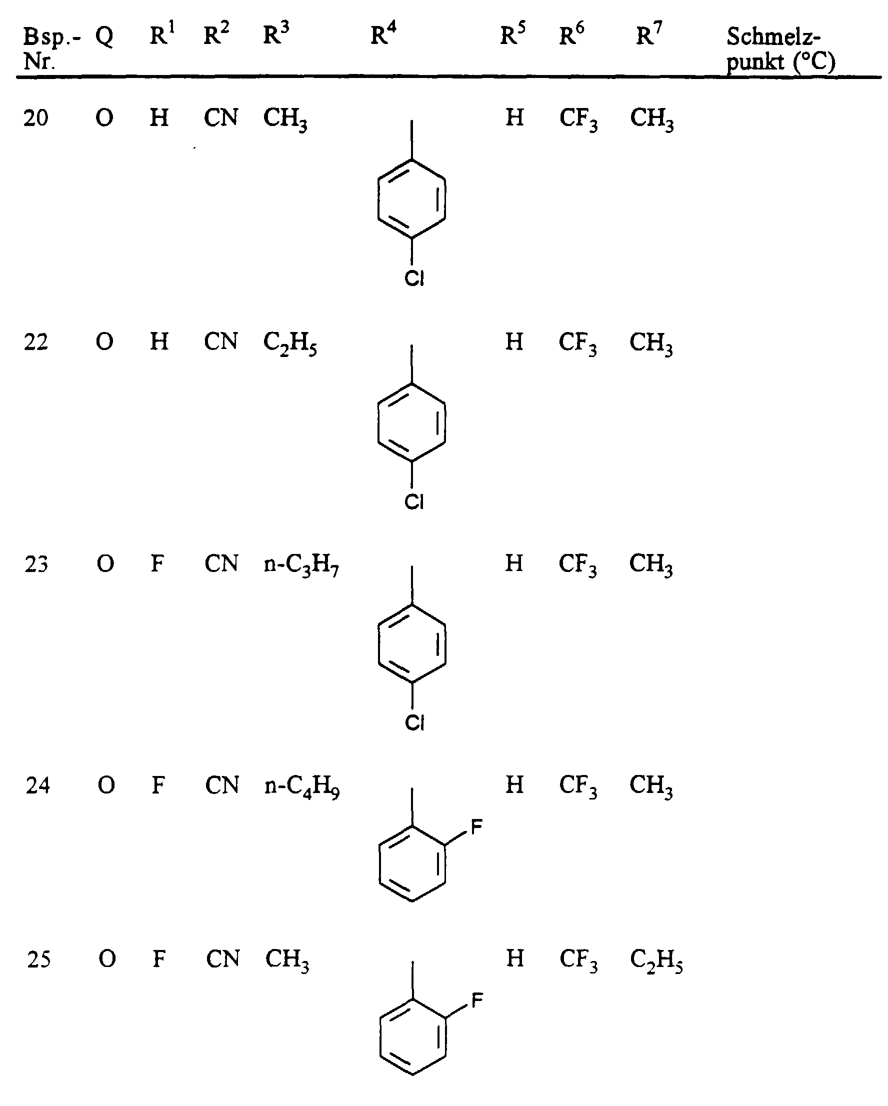

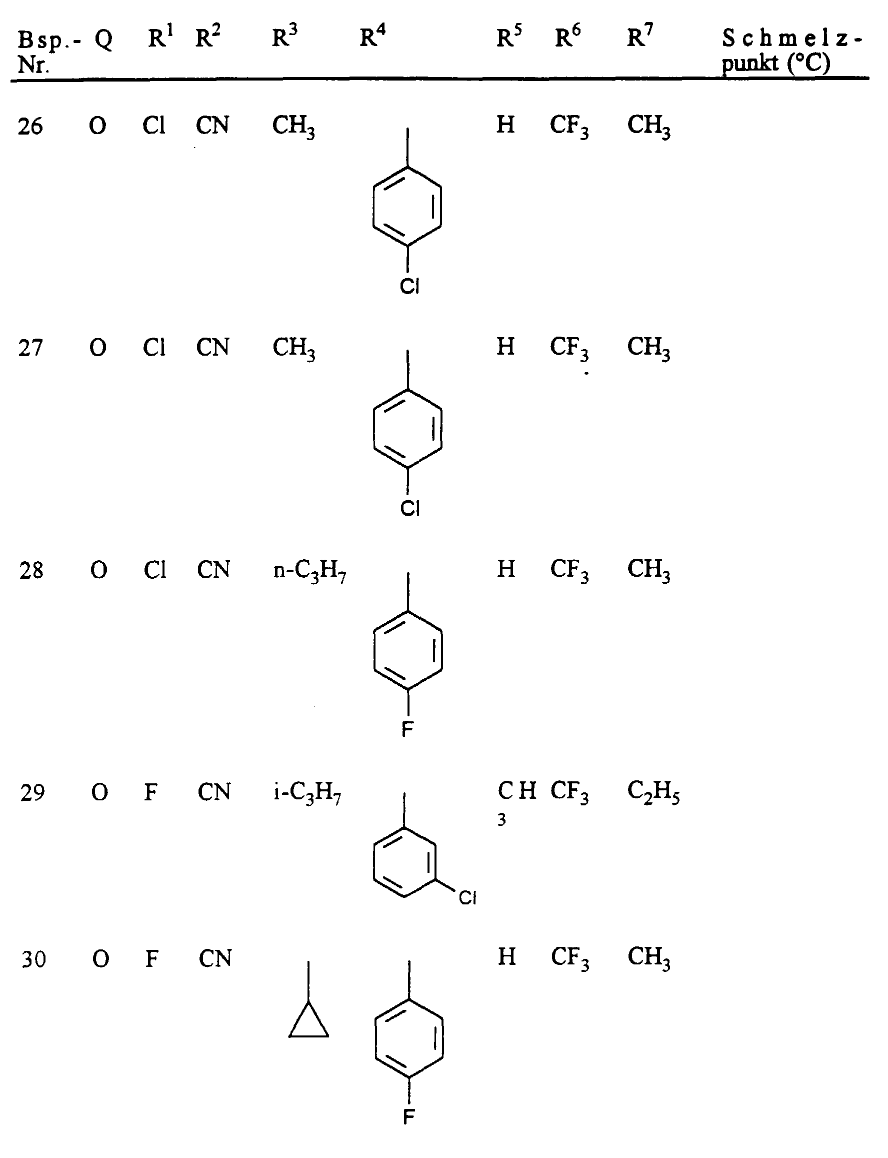

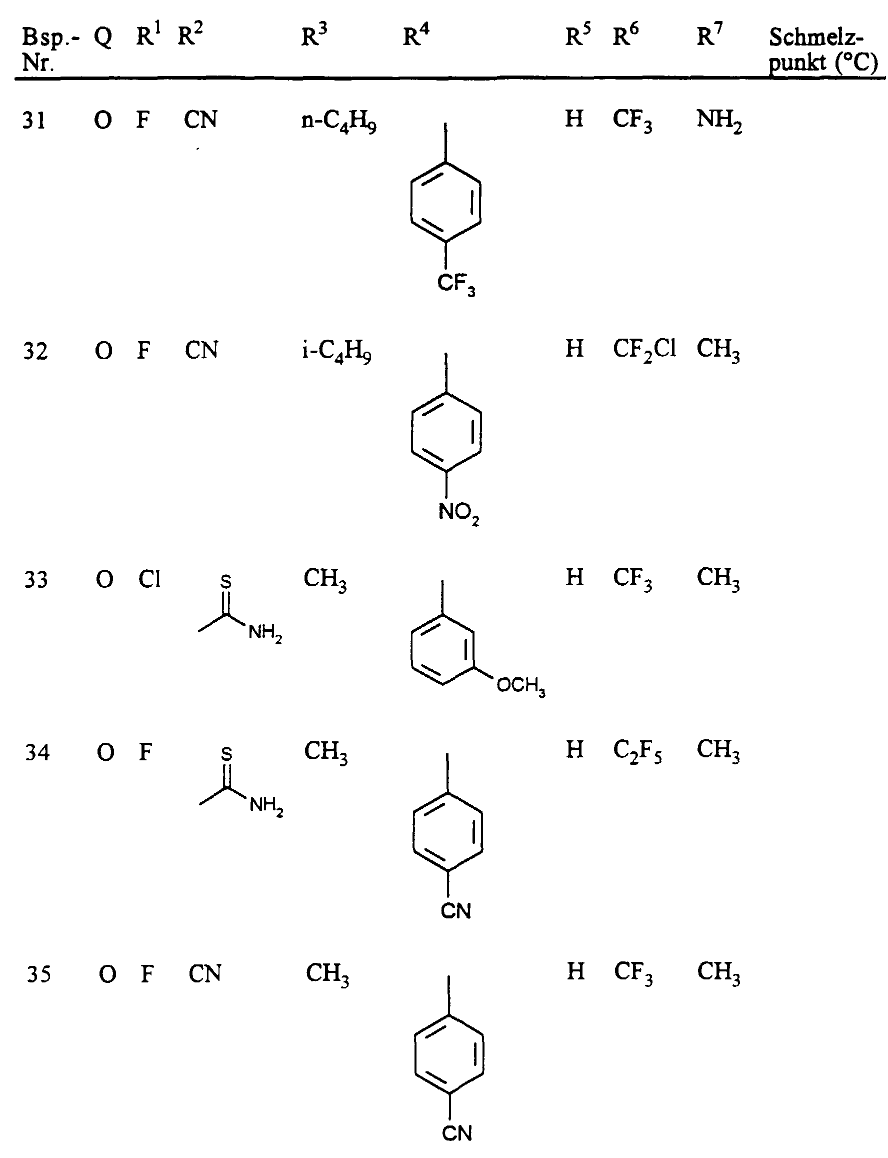

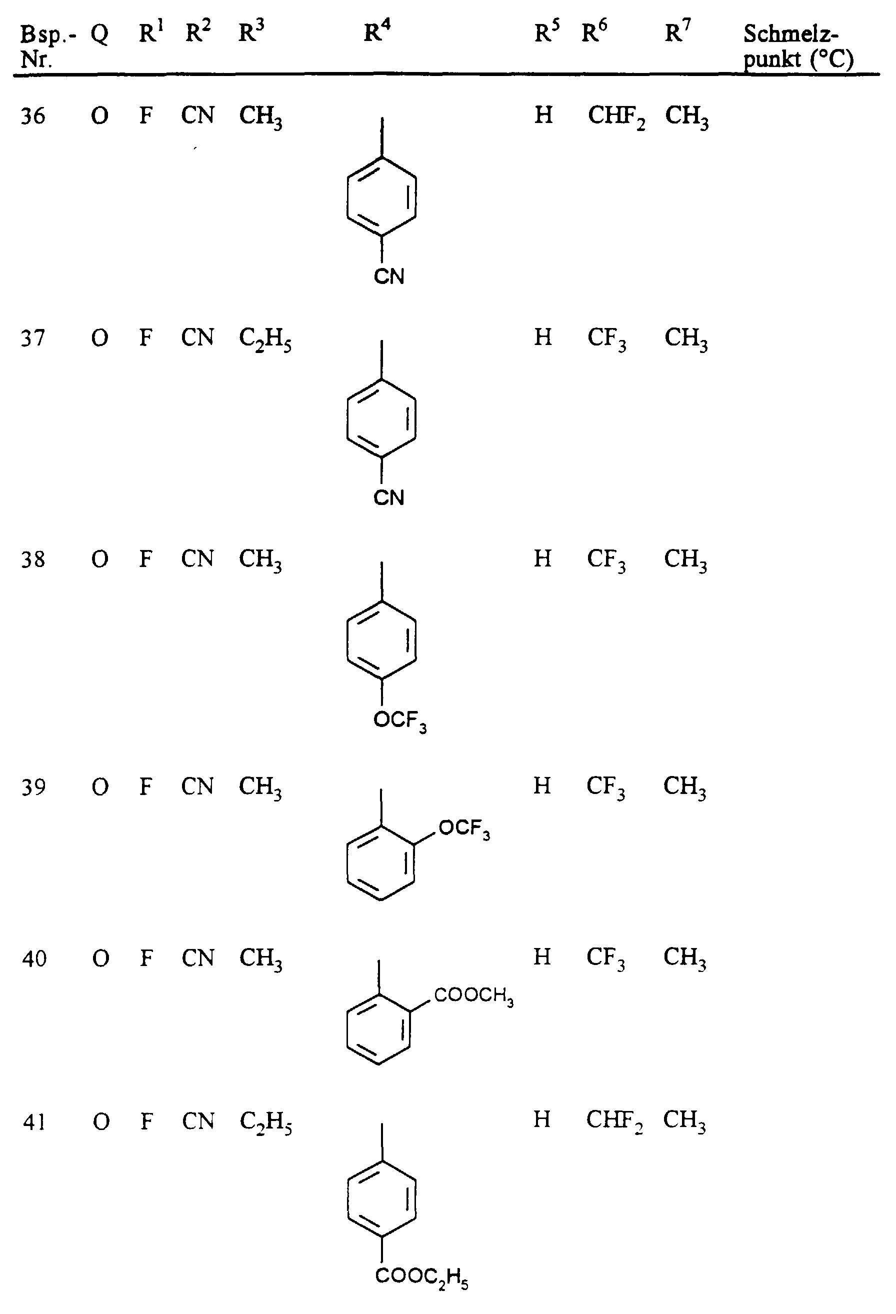

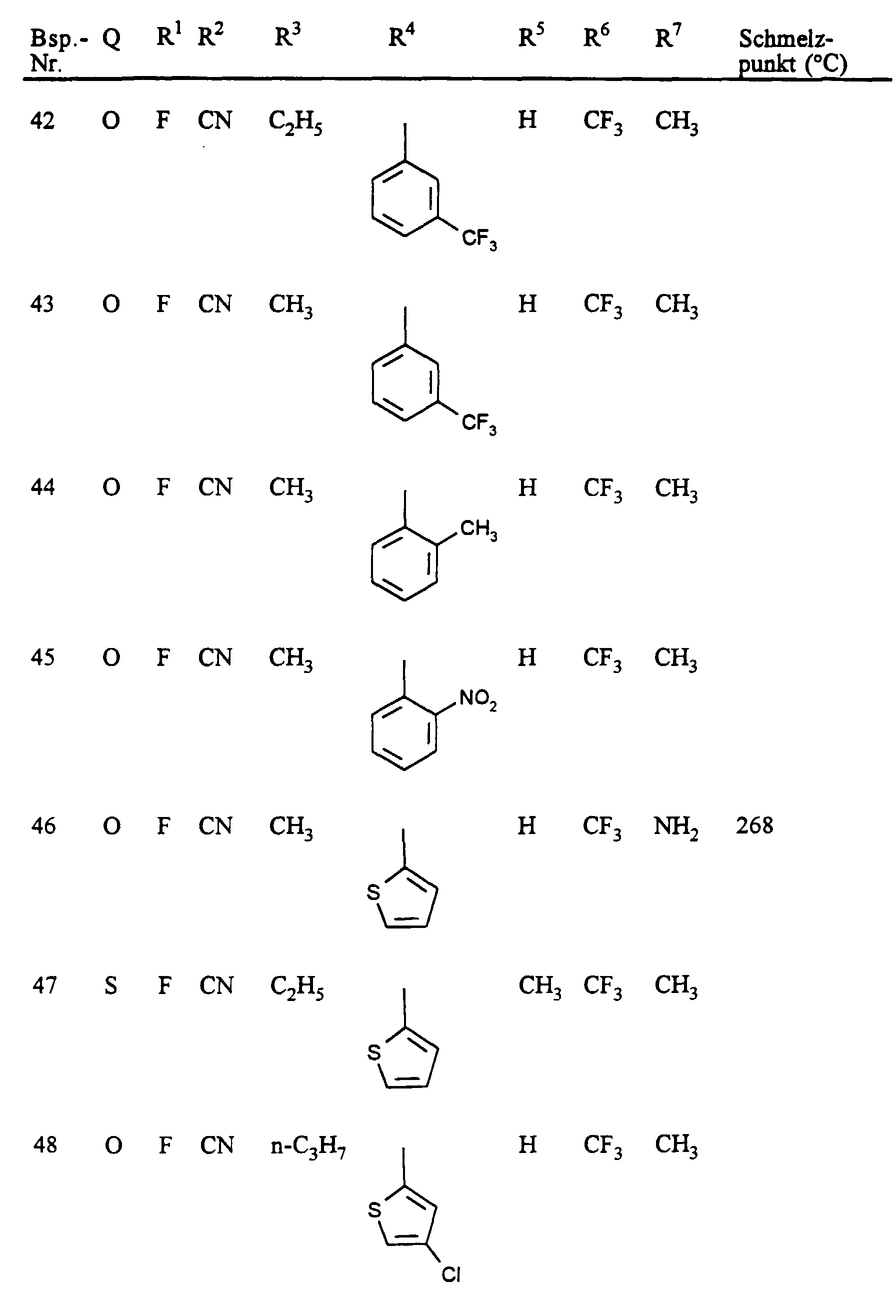

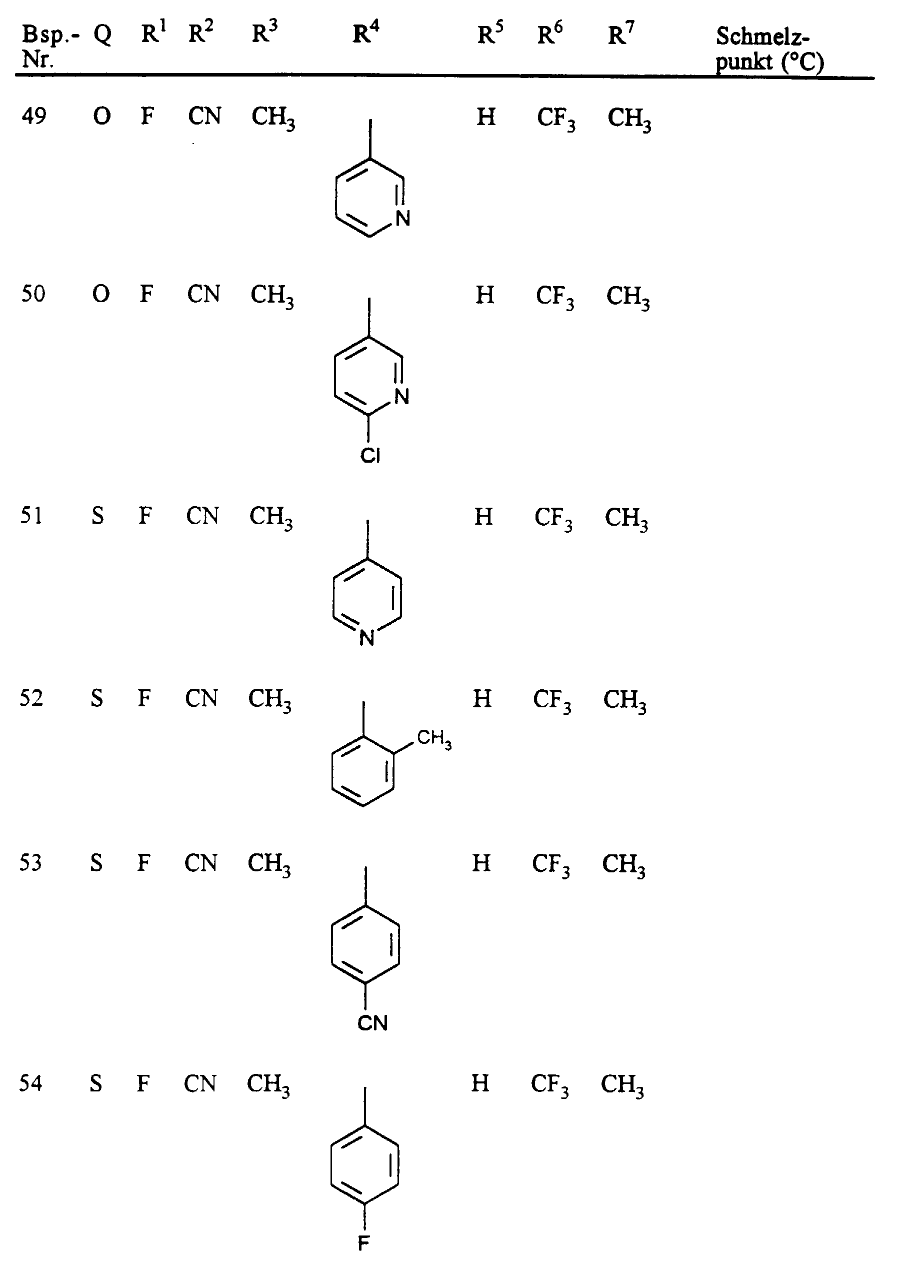

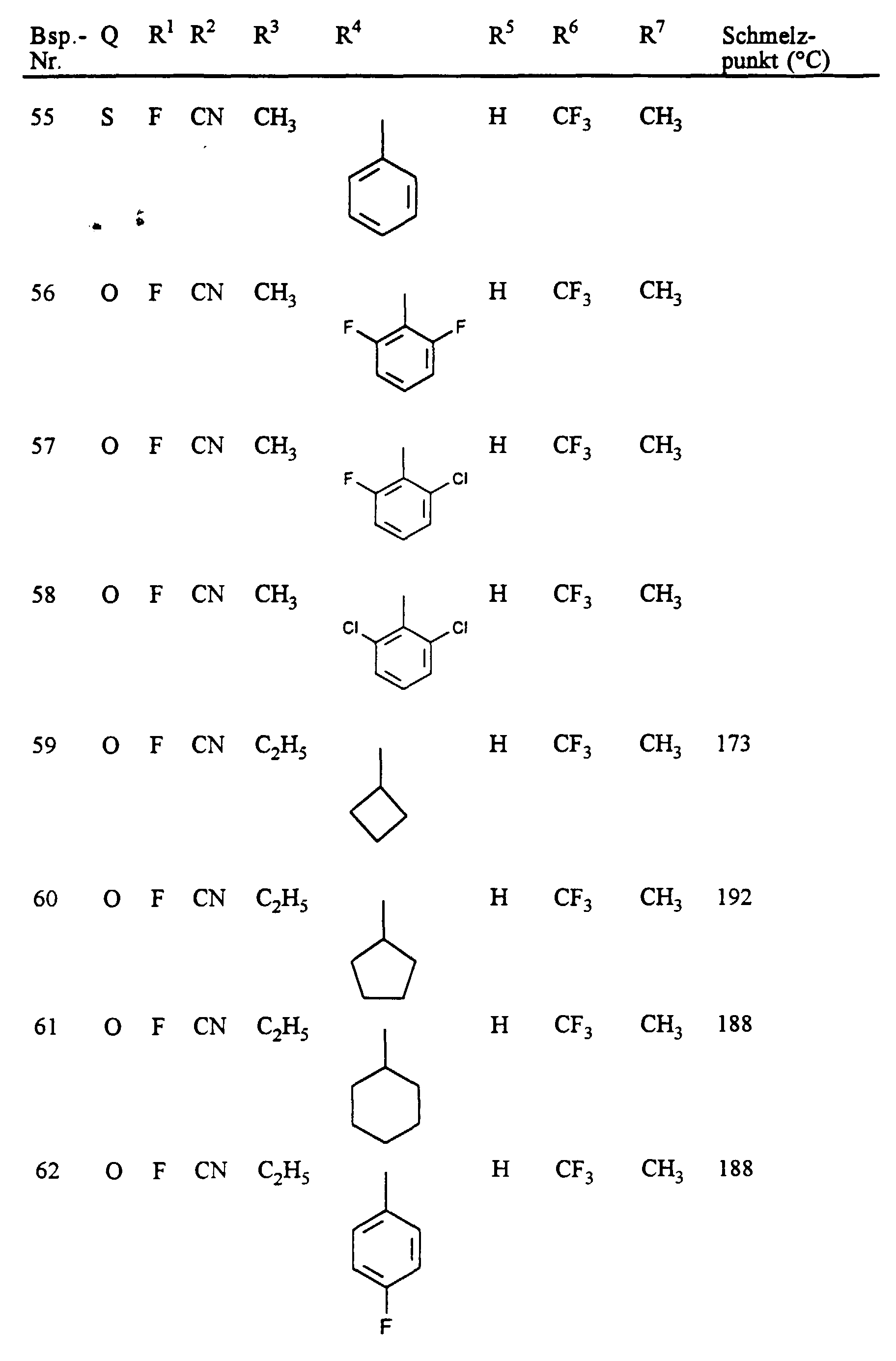

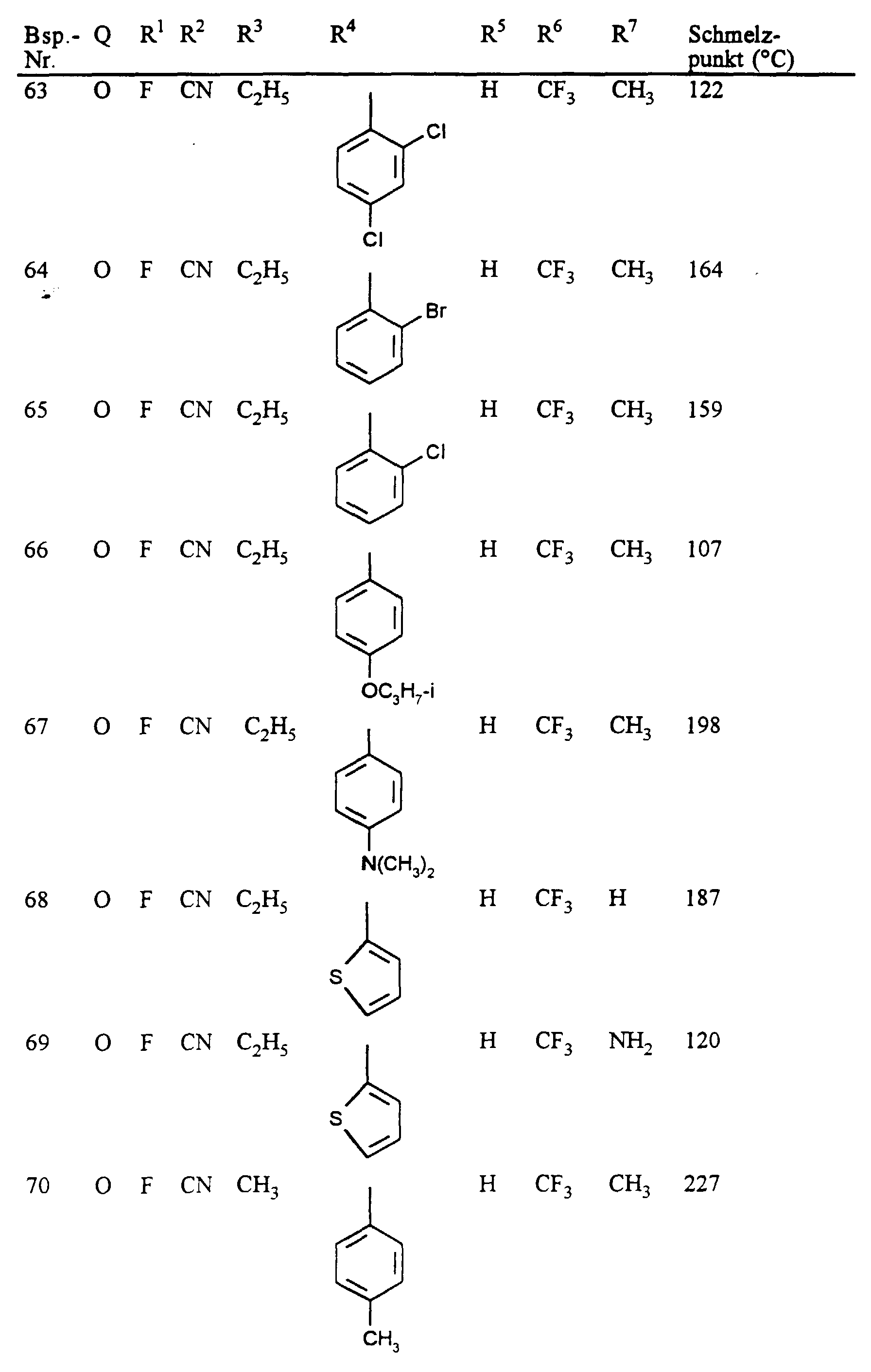

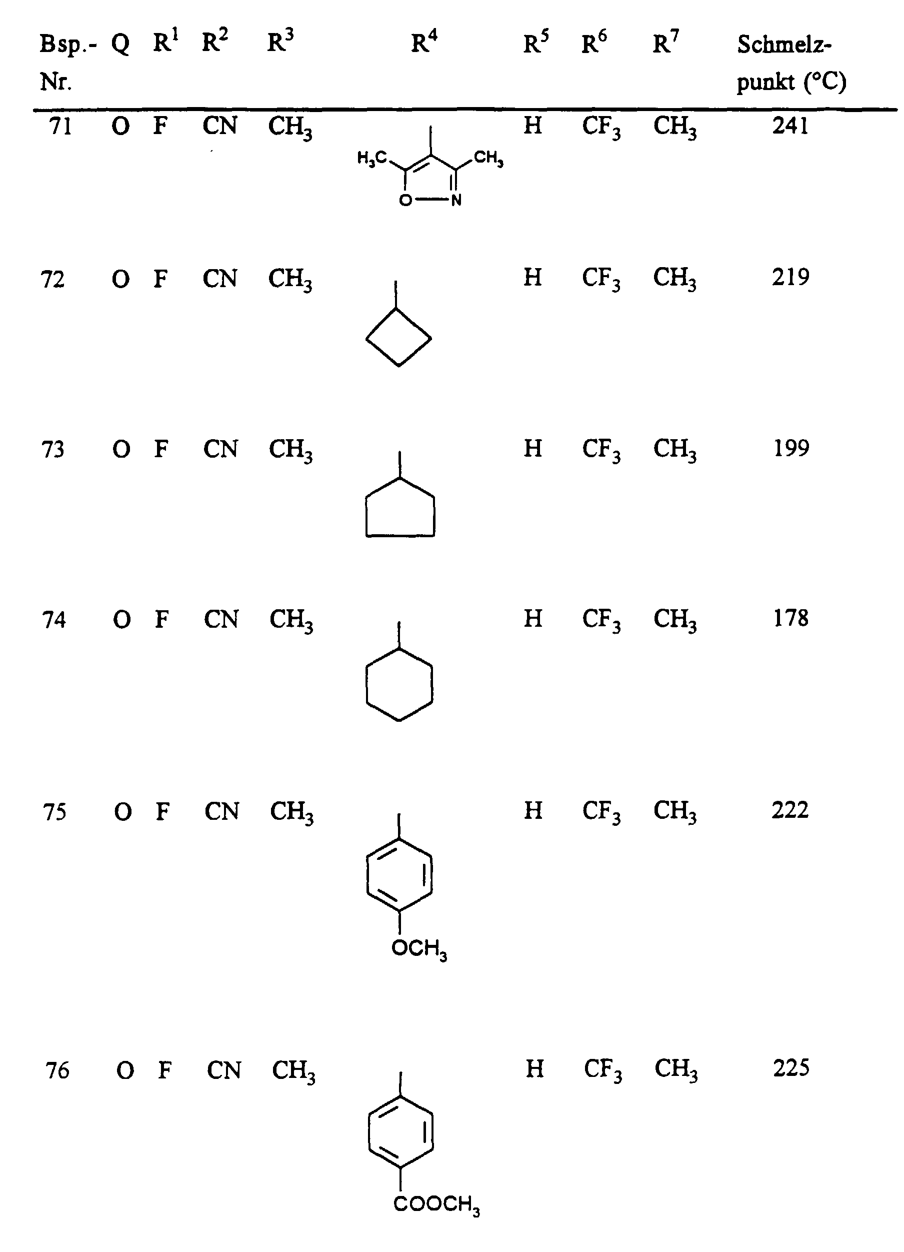

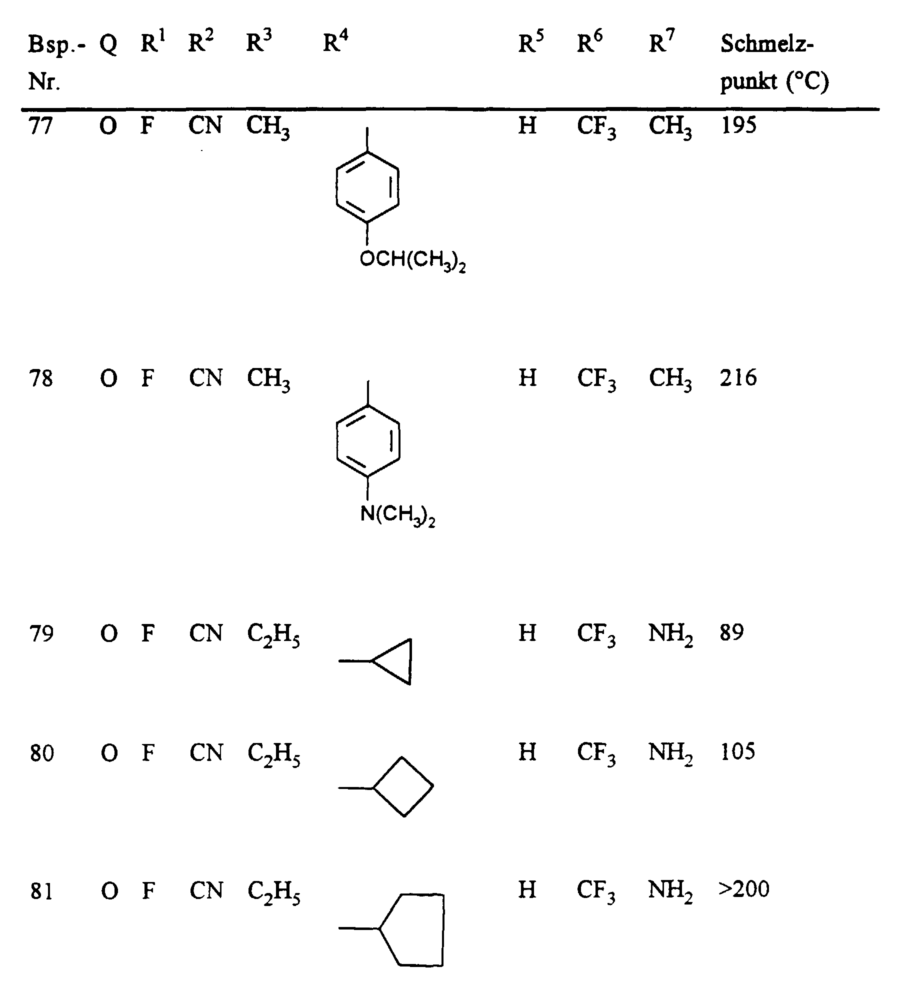

### Anwendungsbeispiele:

### Beispiel A

| Pre-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät. Dieser wird nach 24 Stunden mit der Wirkstoffzubereitung begossen bzw. gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1 und 2 bei sehr guter Verträglichkeit gegenüber Kulturpflanzen, wie z.B. Mais (0 %) und einer Aufwandmenge von 60 g/ha sehr starke Wirkung gegen Unkräuter wie Cyperus (60-100 %), Lolium (70-90 %), Panicum (70-95 %), Abutilon (100 %), Chenopodium (100 %) und Datura (100 %).

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 1 000 l/ha die jeweils gemischten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 1 und 2 bei guter Verträglichkeit gegenüber Kulturpflanzen, wie z.B. Gerste (0-10 %) in einer Aufwandmenge von 30 g/ha sehr starke Wirkung gegen Unkräuter wie Echinochloa (95 %), Sorghum (70-80 %), Abutilon (100 %), Chenopodium (100 %), Datura (100 %) und Solanum (100 %).

### Beispiel C

| Phaedon-Larven-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven Phaedon cochleariae besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z.B. die Verbindungen der Herstellungsbeispiele 1 und 2 bei einer Wirkstoffkonzentration von 0,1 % einen Abtötungsgrad von 80 bis 100 % nach 7 Tagen.

### Beispiel D

| Nephotettix-Test | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade Nephotettix cincticeps besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z.B. die Verbindungen der Herstellungsbeispiele 1 und 2 in einer Wirkstoffkonzentration von 0,1 % einen Abtötungsgrad von 100 % nach 6 Tagen.

## Patentansprüche

1. Substituierte Aminophenyluracile der allgemeinen Formel (I) 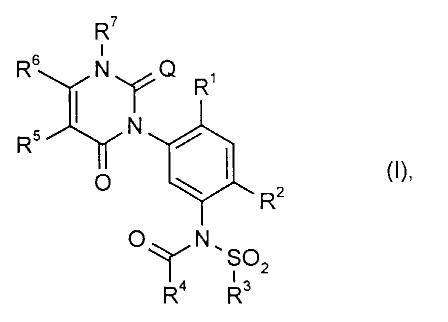 dadurch gekennzeichnet, daß
Q für Sauerstoff oder Schwefel steht,
R' für Wasserstoff, Cyano oder Halogen steht,
R² für Cyano, Thiocarbamoyl oder für gegebenenfalls substituiertes Alkyl steht,
R³ für jeweils gegebenenfalls substituiertes Alkyl, Cycloalkyl, Aryl, Arylalkyl oder Heteroaryl steht,
R⁴ für jeweils gegebenenfalls substituiertes Cycloalkyl, Aryl oder Heteroaryl steht,
R⁵ für Wasserstoff, Halogen oder für jeweils gegebenenfalls substituiertes Alkyl oder Alkoxy steht,
R⁶ für gegebenenfalls substituiertes Alkyl steht und
R⁷ für Wasserstoff, Hydroxy, Amino oder für jeweils gegebenenfalls substituiertes Alkyl, Alkoxy, Alkenyl oder Alkinyl steht, ausgenommen Verbindungen der folgenden Formeln: 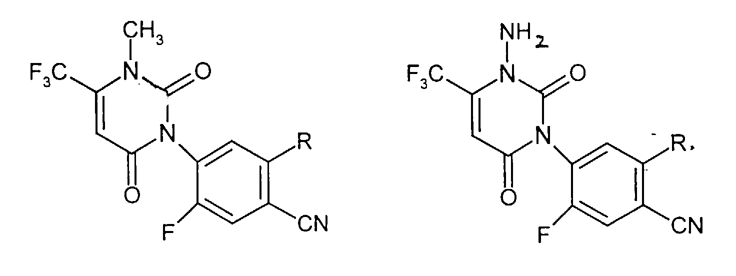
wobei
R für N(SO₂Me)CO(cyclopropyl),
N(SO₂Me)CO Ph,
N(SO₂Et)CO(cyclopropyl) oder
N(SO₂Et)CO Ph steht.

2. Substituierte Aminophenyluracile gemäß Anspruch 1, dadurch gekennzeichnet, daß
Q für Sauerstoff oder Schwefel steht,
R¹ für Wasserstoff, Cyano, Fluor oder Chlor steht,
R² für Cyano, Thiocarbamoyl oder für gegebenenfalls durch Fluor und/oder Chlor substituiertes Alkyl mit 1 bis 4 Kohlenstoffatomen steht,
R³ für gegebenenfalls durch Cyano, Fluor, Chlor, Brom, C₁-C₄-Alkoxy oder C₁-C₄-Alkylthio substituiertes Alkyl mit 1 bis 6 Kohlenstoffatomen steht,
R³ weiterhin für gegebenenfalls durch Cyano, Fluor, Chlor, Brom oder C₁-C₄-Alkyl substituiertes Cycloalkyl mit 3 bis 8 Kohlenstoffatomen steht,
R³ weiterhin für Phenyl, Naphthyl, Benzyl, Phenylethyl, Thienyl, Pyrazolyl, Pyridinyl oder Chinolinyl steht,
wobei als Substituenten jeweils gegebenenfalls in Frage kommen:
Fluor, Chlor, Brom, Cyano, Nitro, Carboxy, Carbamoyl, Thiocarbamoyl, C₁-C₄-Alkyl, C₁-C₄-Alkoxy, C₁-C₄-Alkylthio, Dimethylaminosulfonyl, Diethylaminosulfonyl, jeweils gegebenenfalls durch Fluor und/oder Chlor substituiertes C₁-C₄-Alkylsulfinyl oder C₁-C₄-Alkylsulfonyl, jeweils gegebenenfalls durch Fluor, Chlor, Brom, Cyano, Methoxy oder Ethoxy substituiertes C₁-C₄-Alkoxycarbonyl oder jeweils gegebenenfalls durch Fluor, Chlor, Brom, Cyano, Methyl, Methoxy, Trifluormethyl und/oder Trifluormethoxy substituiertes Phenyl, Phenyloxy oder Phenylthio;
R⁴ für gegebenenfalls durch Cyano, Fluor, Chlor, Brom oder C₁-C₄-Alkyl substituiertes Cycloalkyl mit 3 bis 8 Kohlenstoffatomen steht,
R⁴ weiterhin für jeweils gegebenenfalls substituiertes Phenyl, Naphthyl, Furyl, Thienyl, Oxazolyl, Isoxazolyl, Pyrazolyl, Pyridinyl, Pyrimidinyl oder Chinolinyl steht, wobei als Substituenten jeweils gegebenenfalls in Frage kommen:
Fluor, Chlor, Brom, Cyano, Nitro, Carboxy, Carbamoyl, Thiocarbamoyl, Dimethylamino, Dimethylaminosulfonyl, Diethylaminosulfonyl, jeweils gegebenenfalls durch Fluor und/oder Chlor substituiertes C₁-C₄-Alkyl, C₁-C₄-Alkoxy, C₁-C₄-Alkylthio, C₁-C₄-Alkylsulfinyl oder C₁-C₄-Alkylsulfonyl oder gegebenenfalls durch Fluor, Chlor, Brom, Cyano, Methoxy oder Ethoxy substituiertes C₁-C₄-Alkoxycarbonyl;
R⁵ für Wasserstoff, Fluor, Chlor, Brom oder für jeweils gegebenenfalls durch Fluor und/oder Chlor substituiertes Alkyl oder Alkoxy mit jeweils 1 bis 4 Kohlenstoffatomen steht,
R⁶ für gegebenenfalls durch Fluor und/oder Chlor substituiertes Alkyl mit 1 bis 4 Kohlenstoffatomen steht und
R⁷ für Wasserstoff, Hydroxy, Amino oder für jeweils gegebenenfalls durch Fluor, Chlor oder C₁-C₄-Alkoxy substituiertes Alkyl, Alkoxy, Alkenyl oder Alkinyl mit jeweils bis zu 6 Kohlenstoffatomen steht.

3. Substituierte Aminophenyluracile gemäß Anspruch 1, dadurch gekennzeichnet, daß
Q für Sauerstoff oder Schwefel steht,
R¹ für Wasserstoff, Cyano, Fluor oder Chlor steht,
R² für Cyano, Thiocarbamoyl, Methyl, Ethyl, n- oder i-Propyl, n-, i-, s- oder t-Butyl oder Trifluormethyl steht,
R³ für jeweils gegebenenfalls durch Cyano, Fluor, Chlor, Methoxy oder Ethoxy substituiertes Methyl, Ethyl, n- oder i-Propyl, n-, i-, s- oder t-Butyl steht,
R³ weiterhin für jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl steht,
R³ weiterhin für jeweils gegebenenfalls substituiertes Phenyl, Naphthyl, Benzyl, Phenylethyl, Thienyl, Pyrazolyl, Pyridinyl oder Chinolinyl steht,
wobei als Substituenten in Frage kommen: Fluor, Chlor, Brom, Cyano, Nitro, Carboxy, Carbamoyl, Thiocarbamoyl, Methyl, Ethyl, n- oder i-Propyl, Trifluormethyl, Methoxy, Ethoxy, n- oder i-Propoxy, Difluormethoxy, Trifluormethoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, Dimethylaminosulfonyl, Diethylaminosulfonyl, Methoxycarbonyl, Ethoxycarbonyl, n- oder i-Propoxycarbonyl,
R⁴ für gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl steht,
R⁴ weiterhin für jeweils gegebenenfalls substituiertes Phenyl, Naphthyl, Furyl, Thienyl, Oxazolyl, Isoxazolyl, Pyrazolyl, Pyridinyl oder Pyrimidinyl steht, wobei als Substituenten jeweils gegebenenfalls in Frage kommen: Fluor, Chlor, Brom, Cyano, Nitro, Carboxy, Carbamoyl, Thiocarbamoyl, Dimethylamino, Dimethylaminosulfonyl oder Diethylaminosulfonyl, jeweils gegebenenfalls durch Fluor und/oder Chlor substituiertes Methyl, Ethyl, n- oder i-Propyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, jeweils gegebenenfalls durch Fluor, Chlor, Brom, Cyano, Methoxy oder Ethoxy substituiertes Methoxy-carbonyl, Ethoxycarbonyl, n- oder i-Propoxycarbonyl, jeweils gegebenenfalls durch Fluor, Chlor, Brom, Cyano, Methyl, Methoxy, Trifluormethyl und/oder Trifluormethoxy substituiertes Phenyl, Phenyloxy oder Phenylthio,
R⁵ für Wasserstoff, Fluor, Chlor, Brom oder für jeweils gegebenenfalls durch Fluor und/oder Chlor substituiertes Methyl, Ethyl, n- oder i-Propyl steht,
R⁶ für gegebenenfalls durch Fluor und/oder Chlor substituiertes Methyl, Ethyl, n- oder i-Propyl steht und
R⁷ für Wasserstoff, Amino oder für jeweils gegebenenfalls durch Fluor und/oder Chlor substituiertes Methyl, Ethyl, n- oder i-Propyl, n-, i- oder s-Butyl, Methoty, Ethoxy, n- oder i-Propoxy, n-, i- oder s-Butoxy, Propenyl, Butenyl, Propinyl oder Butinyl steht.

4. Verfahren zur Herstellung substituierter Aminophenyluracile gemäß einem der Ansprüche 1 bis 3, dadurch gekennzeichnet, daß Sulfonylaminophenyluracile der allgemeinen Formel (II) 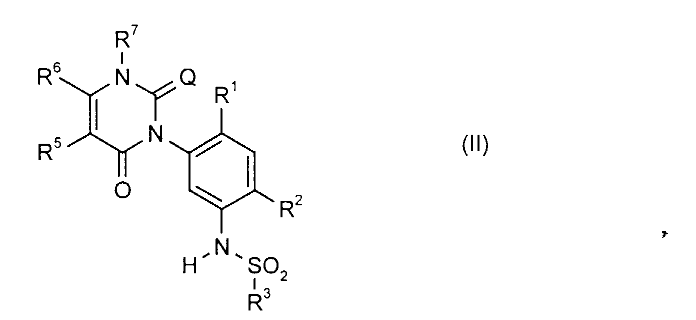 in welcher
Q, R¹, R², R³, R⁵, R⁶ und R⁷ die in einem der Ansprüche 1 bis 3 angegebenen Bedeutungen haben,
mit Säurederivaten der allgemeinen Formel (III)
R⁴-CO-X (III)
in welcher
R⁴ die in einem der Ansprüche 1 bis 3 angegebene Bedeutung hat und
X für Halogen oder die Gruppierung -O-CO-R⁴ steht,
gegebenenfalls in Gegenwart eines Reaktionshilfsmittels und gegebenenfalls in Gegenwart eines Verdünnungsmittels umsetzt.

5. Verfahren zur Bekämpfung von unerwünschten Pflanzen, dadurch gekennzeichnet, daß man substituierte Aminophenyluracile gemäß einem der Ansprüche 1 bis 3 auf Pflanzen und/oder ihren Lebensraum einwirken läßt.

6. Verwendung von substituierten Aminophenyluracilen gemäß einem der Ansprüche 1 bis 3 zur Bekämpfung von unerwünschten Pflanzen und unerwünschten Insekten.

7. Verfahren zur Herstellung von herbiziden und insektiziden Mitteln, dadurch gekennzeichnet, daß man substituierte Aminophenyluracile gemäß einem der Ansprüche 1 bis 3 mit Streckmitteln und/oder oberflächenaktiven Mitteln vermischt.

8. Herbizide Mittel und insektizide Mittel, gekennzeichnet durch einen Gehalt an mindestens einem substituierten Aminophenyluracil gemäß einem der Ansprüche 1 bis 3.

9. Verfahren zur Bekämpfung unerwünschter Insekten, dadurch gekennzeichnet, daß man substituierte Aminophenyluracile gemäß einem der Ansprüche 1 bis 3 auf Insekten und/oder deren Lebensraum einwirken läßt.

## Claims

1. Substituted aminophenyluracils of the general formula (I) 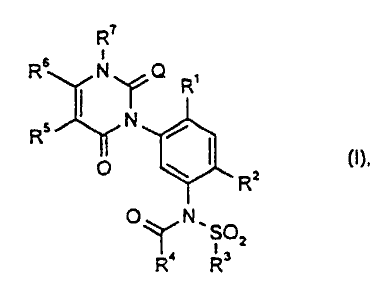 characterized in that
Q represents oxygen or sulphur,
R¹ represents hydrogen, cyano or halogen,
R² represents cyano, thiocarbamoyl, or represents optionally substituted alkyl,
R³ represents respectively optionally substituted alkyl, cycloalkyl, aryl, arylalkyl or heteroaryl,
R⁴ represents respectively optionally substituted cycloalkyl, aryl or heteroaryl,
R⁵ represents hydrogen, halogen or represents respectively optionally substituted alkyl or alkoxy,
R⁶ represents optionally substituted alkyl and
R⁷ represents hydrogen, hydroxyl, amino or represents respectively optionally substituted alkyl, alkoxy, alkenyl or alkinyl , except for compounds of the following formulae: 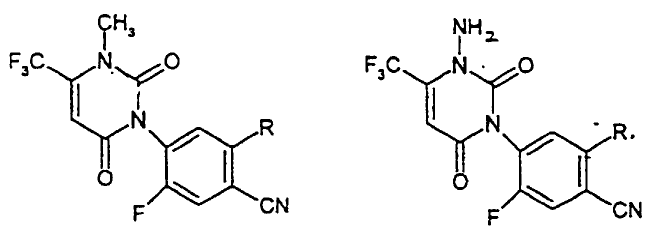
where
R represents
N(SO₂Me)CO(cyclopropyl),
N(SO₂Me)CO Ph,
N(SO₂Et)CO(cyclopropyl) or
N(SO₂Et)CO Ph

2. Substituted aminophenyluracils according to Claim 1, characterized in that
Q represents oxygen or sulphur,
R¹ represents hydrogen, cyano, fluorine or chlorine,
R² represents cyano, thiocarbamoyl, or represents optionally fluorine- and/or chlorine-substituted alkyl having 1 to 4 carbon atoms,
R³ represents optionally cyano-, fluorine-, chlorine-, bromine-, C₁-C₄-alkoxy- or C₁-C₄-alkylthio-substituted alkyl having 1 to 6 carbon atoms,
R³ furthermore represents optionally cyano-, fluorine-, chlorine-, bromine- or C₁-C₄-alkyl-substituted cycloalkyl having 3 to 8 carbon atoms,
R³ furthermore represents phenyl, naphthyl, benzyl, phenylethyl, thienyl, pyrazolyl, pyridinyl or quinolinyl,
possible substituents in each case being:
fluorine, chlorine, bromine, cyano, nitro, carboxy, carbamoyl, thiocarbamoyl, C₁-C₄-alkyl, C₁-C₄-alkoxy, C₁-C₄-alkylthio, dimethylaminosulphonyl, diethylaminosulphonyl, respectively optionally fluorine- and/or chlorine-substituted C₁-C₄-alkylsulphinyl or C₁-C₄-alkylsulphonyl, respectively optionally fluorine-, chlorine-, bromine-, cyano-, methoxy- or ethoxy-substituted C₁-C₄-alkoxycarbonyl or respectively optionally fluorine-, chlorine-, bromine-, cyano-, methyl-, methoxy-, trifluoromethyl- and/or trifluoromethoxy-substituted phenyl, phenyloxy or phenylthio;
R⁴ represents optionally cyano-, fluorine-, chlorine-, bromine- or C₁-C₄-alkyl-substituted cycloalkyl having 3 to 8 carbon atoms,
R⁴ furthermore represents respectively optionally substituted phenyl, naphthyl, furyl, thienyl, oxazolyl, isoxazolyl, pyrazolyl, pyridinyl, pyrimidinyl or quinolinyl, possible substituents in each case being:
fluorine, chlorine, bromine, cyano, nitro, carboxy, carbamoyl, thiocarbamoyl, dimethylamino, dimethylaminosulphonyl, diethylaminosulphonyl, respectively optionally fluorine- and/or chlorine-substituted C₁-C₄-alkyl, C₁-C₄-alkoxy,C₁-C₄-alkylthio, C₁-C₄-alkylsulphinyl or C₁-C₄-alkylsulphonyl or optionally fluorine-, chlorine-, bromine-, cyano-, methoxy- or ethoxy-substituted C₁-C₄-alkoxycarbonyl;
R⁵ represents hydrogen, fluorine, chlorine, bromine or represents respectively optionally fluorine- and/or chlorine-substituted alkyl or alkoxy having in each case 1 to 4 carbon atoms,
R⁶ represents optionally fluorine- and/or chlorine-substituted alkyl having 1 to 4 carbon atoms and
R⁷ represents hydrogen, hydroxyl, amino or represents respectively optionally fluorine-, chlorine- or C₁-C4-alkoxy-substituted alkyl, alkoxy, alkenyl or alkinyl having in each case up to 6 carbon atoms.

3. Substituted aminophenyluracils according to Claim 1, characterized in that
Q represents oxygen or sulphur,
R¹ represents hydrogen, cyano, fluorine or chlorine,
R² represents cyano, thiocarbamoyl, methyl, ethyl, n- or i-propyl, n-, i-, s- or t-butyl or trifluoromethyl,
R³ represents respectively optionally cyano-, fluorine-, chlorine-, methoxy- or ethoxy-substituted methyl, ethyl, n- or i-propyl, n-, i-, s- or t-butyl,
R³ furthermore represents respectively optionally cyano-, fluorine-, chlorine-, bromine-, methyl-, ethyl-, n- or i-propyl-substituted cyclopropyl, cyclobutyl, cyclopentyl or cyclohexyl,
R³ furthermore represents respectively optionally substituted phenyl, naphthyl, benzyl, phenylethyl, thienyl, pyrazolyl, pyridinyl or quinolinyl, possible substituents being: fluorine, chlorine, bromine, cyano, nitro, carboxy, carbamoyl, thiocarbamoyl, methyl, ethyl, n- or i-propyl, trifluoromethyl, methoxy, ethoxy, n- or i-propoxy, difluoromethoxy, trifluoromethoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, dimethylaminosulphonyl, diethylaminosulphonyl, methoxycarbonyl, ethoxycarbonyl, n- or i-propoxycarbonyl,
R⁴ represents optionally cyano-, fluorine-, chlorine-, bromine-, methyl-, ethyl-, n- or i-propyl-substituted cyclopropyl, cyclobutyl, cyclopentyl or cyclohexyl,
R⁴ furthermore represents respectively optionally substituted phenyl, naphthyl, furyl, thienyl, oxazolyl, isoxazolyl, pyrazolyl, pyridinyl or pyrimidinyl, possible substituents in each case being: fluorine, chlorine, bromine, cyano, nitro, carboxy, carbamoyl, thiocarbamoyl, dimethylamino, dimethylaminosulphonyl or diethylaminosulphonyl, respectively optionally fluorine- and/or chlorine-substituted methyl, ethyl, n- or i-propyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, respectively optionally fluorine-, chlorine-, bromine-, cyano-, methoxy- or ethoxy-substituted methoxycarbonyl, ethoxycarbonyl, n- or i-propoxycarbonyl, respectively optionally fluorine-, chlorine-, bromine-, cyano-, methyl-, methoxy-, trifluoromethyl- and/or trifluoromethoxy-substituted phenyl, phenyloxy or phenylthio,
R⁵ represents hydrogen, fluorine, chlorine, bromine or represents respectively optionally fluorine- and/or chlorine-substituted methyl, ethyl, n- or i-propyl,
R⁶ represents optionally fluorine- and/or chlorine-substituted methyl, ethyl, n- or i-propyl and
R⁷ represents hydrogen, amino or represents respectively optionally fluorine- and/or chlorine-substituted methyl, ethyl, n- or i-propyl, n-, i- or s-butyl, methoxy, ethoxy, n- or i-propoxy, n-, i- or s-butoxy, propenyl, butenyl, propinyl or butinyl.

4. Process for preparing substituted aminophenyluracils according to one of Claims 1 to 3,
characterized in that sulphonylaminophenyluracils of the general formula (II) 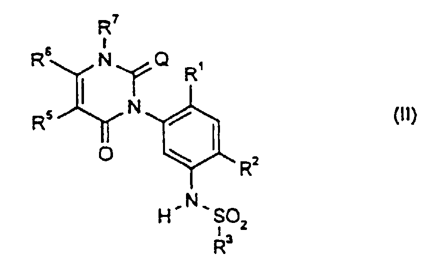 in which
Q, R¹, R², R³, R⁴, R⁵, R⁶ and R⁷ are each as defined in one of Claims 1 to 3,
are reacted with acid derivatives of the general formula (III)
R⁴-CO-X (III)
in which
R⁴ is as defined in one of Claims 1 to 3, and
X represents halogen or the grouping -O-CO-R⁴,
if appropriate in the presence of a reaction auxiliary and if appropriate in the presence of a diluent.

5. Method for controlling undesirable plants, characterized in that substituted aminophenyluracils according to one of Claims 1 to 3 are allowed to act on plants and/or their habitat.

6. Use of substituted aminophenyluracils according to one of Claims 1 to 3 for controlling undesirable plants and undesirable insects.

7. Process for preparing herbicides and insecticides, characterized in that substituted aminophenyluracils according to one of Claims 1 to 3 are mixed with extenders and/or surfactants.

8. Herbicides and insecticides, characterized in that they comprise at least one substituted aminophenyluracil according to one of Claims 1 to 3.

9. Method for controlling undesirable insects, characterized in that substituted aminophenyluracils according to one of Claims 1 to 3 are allowed to act on insects and/or their habitat.

## Revendications

1. Aminophényluraciles substitués de formule générale (I) 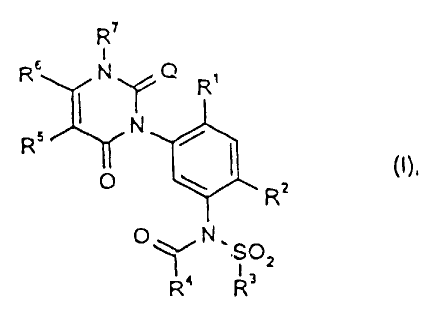 caractérisés en ce que
Q représente l'oxygène ou le soufre,
R¹ est l'hydrogène, un groupe cyano ou un halogène,
R² est un groupe cyano, thiocarbamoyle ou un groupe alkyle éventuellement substitué,
R³ est un groupe alkyle, cycloalkyle, aryle, arylalkyle ou hétéroaryle éventuellement substitué dans chaque cas,
R⁴ est un groupe cycloalkyle, aryle ou hétéroaryle éventuellement substitué dans chaque cas,
R⁵ est l'hydrogène, un halogène ou un groupe alkyle ou alkoxy éventuellement substitué dans chaque cas,
R⁶ est un groupe alkyle éventuellement substitué et
R⁷ est l'hydrogène, un groupe hydroxy, amino ou un groupe alkyle, alkoxy, alcényle ou alcynyle éventuellement substitué dans chaque cas, excepté les composés de formules suivantes : 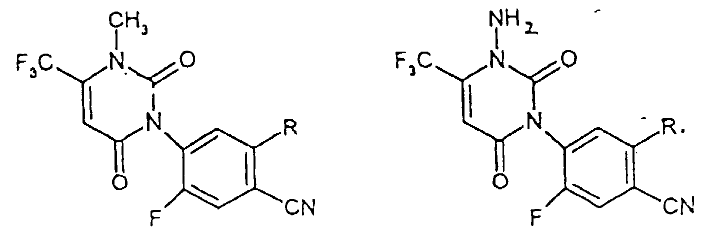
dans lesquelles
R représente un groupe N(SO₂Me)CO(cyclopropyle),
N(SO₂Me)CO Ph,
N(SO₂Et)CO(cyclopropyle) ou
N(SO₂Et)CO Ph.

2. Aminophényluraciles substitués suivant la revendication 1, caractérisés en ce que
Q est l'oxygène ou le soufre,
R¹ est l'hydrogène, un groupe cyano, le fluor ou le chlore,
R² est un groupe cyano, thiocarbamoyle ou un groupe alkyle ayant 1 à 4 atomes de carbone, éventuellement substitué par du fluor et/ou du chlore,
R³ est un groupe alkyle ayant 1 à 6 atomes de carbone, éventuellement substitué par un radical cyano, du fluor, du chlore, du brome, un radical alkoxy en C₁ à C₄ ou alkylthio en C₁ à C₄,
R³ représente en outre un groupe cycloalkyle de 3 à 8 atomes de carbone, éventuellement substitué par un radical cyano, du fluor, du chlore, du brome ou un radical alkyle en C₁ à C₄,
R³ est en outre un groupe phényle, naphtyle, benzyle, phényléthyle, thiényle, pyrazolyle, pyridinyle ou quinolinyle,
en considérant éventuellement dans chaque cas comme substituants :
fluor, chlore, brome, cyano, nitro, carboxy, carbamoyle, thiocarbamoyle, alkyle en C₁ à C₄, alkoxy en C₁ à C₄, alkylthio en C₁ à C₄, diméthylaminosulfonyle, diéthylaminosulfonyle, alkylsulfinyle en C₁ à C₄ ou alkylsulfonyle en C₁ à C₄ dont chacun est éventuellement substitué par du fluor et/ou du chlore, (alkoxy en C₁ à C₄)carbonyle éventuellement substitué dans chaque cas par du fluor, du chlore, du brome, un radical cyano, méthoxy ou éthoxy, ou phényle, phényloxy ou phénylthio dont chacun est éventuellement substitué par du fluor, du chlore, du brome, un radical cyano, méthyle, méthoxy, trifluorométhyle et/ou trifluorométhoxy ;
R⁴ est un groupe cycloalkyle de 3 à 8 atomes de carbone, éventuellement substitué par un radical cyano, fluoro, chloro, bromo ou alkyle en C₁ à C₄,
R⁴ représente en outre un groupe phényle, naphtyle, furyle, thiényle, oxazolyle, isoxazolyle, pyrazolyle, pyridinyle, pyrimidinyle ou quinolinyle dont chacun est éventuellement substitué, en considérant éventuellement dans chaque cas comme substituants :
fluor, chlore, brome, cyano, nitro, carboxy, carbamoyle, thiocarbamoyle, diméthylamino, diméthylaminosulfonyle, diéthylaminosulfonyle, alkyle en C₁ à C₄, alkoxy en C₁ à C₄, alkylthio en C₁ à C₄, alkylsulfinyle en C₁ à C₄ ou alkylsulfonyle en C₁ à C₄ dont chacun est éventuellement substitué par du fluor et/ou du chlore, ou (alkoxy en C₁ à C₄)carbonyle éventuellement substitué par du fluor, du chlore, du brome, un radical cyano, méthoxy ou éthoxy,
R⁵ représente l'hydrogène, le fluor, le chlore, le brome ou un groupe alkyle ou alkoxy ayant chacun 1 à 4 atomes de carbone et dont chacun est éventuellement substitué par du fluor et/ou du chlore,
R⁶ est un groupe alkyle ayant 1 à 4 atomes de carbone éventuellement substitué par du fluor et/ou du chlore ; et
R⁷ est l'hydrogène, un groupe hydroxy, amino ou un groupe alkyle, alkoxy, alcényle ou alcynyle ayant chacun jusqu'à 6 atomes de carbone et dont chacun est éventuellement substitué par du fluor, du chlore ou un radical alkoxy en C₁ à C₄.

3. Aminophényluraciles substitués suivant la revendication 1, caractérisés en ce que :
Q est l'oxygène ou le soufre,
R¹ est l'hydrogène, un groupe cyano, le fluor ou le chlore,
R² est un groupe cyano, thiocarbamoyle, méthyle, éthyle, n-propyle, isopropyle, n-butyle, isobutyle, sec.-butyle, tertio-butyle ou trifluorométhyle,
R³ est un groupe méthyle, éthyle, n-propyle, isopropyle, n-butyle, isobutyle, sec.-butyle ou tertio-butyle éventuellement substitué dans chaque cas par un radical cyano, le fluor, le chlore, un radical méthoxy ou éthoxy,
R³ représente en outre un groupe cyclopropyle, cyclobutyle, cyclopentyle ou cyclohexyle dont chacun est éventuellement substitué par un radical cyano, du fluor, du chlore, du brome, un radical méthyle, éthyle, n-propyle ou isopropyle,
R³ représente en outre un groupe phényle, naphtyle, benzyle, phényléthyle, thiényle, pyrazolyle, pyridinyle ou quinolinyle dont chacun est éventuellement substitué,
en considérant comme substituants : fluor, chlore, brome, cyano, nitro, carboxy, carbamoyle, thiocarbamoyle, méthyle, éthyle, n-propyle, isopropyle, trifluorométhyle, méthoxy, éthoxy, n-propoxy, isopropoxy, difluorométhoxy, trifluorométhoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, diméthylaminosulfonyle, diéthylaminosulfonyle, méthoxycarbonyle, éthoxycarbonyle, n-propoxycarbonyle ou isopropoxycarbonyle,
R⁴ est un groupe cyclopropyle, cyclobutyle, cyclopentyle ou cyclohexyle portant éventuellement un substituant cyano, fluoro, chloro, bromo, méthyle, éthyle, n-propyle ou isopropyle,
R⁴ représente en outre un groupe phényle, naphtyle, furyle, thiényle, oxazolyle, isoxazolyle, pyrazolyle, pyridinyle ou pyrimidinyle dont chacun est éventuellement substitué, en considérant éventuellement comme substituants dans chaque cas :
fluor, chlore, brome, cyano, nitro, carboxy, carbamoyle, thiocarbamoyle, diméthylamino, diméthylaminosulfonyle ou diéthylaminosulfonyle, méthyle, éthyle, n-propyle, isopropyle, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle, dont chacun est éventuellement substitué par du fluor et/ou du chlore, méthoxycarbonyle, éthoxycarbonyle, n-propoxycarbonyle, isopropoxycarbonyle, dont chacun est éventuellement substitué par du fluor, du chlore, du brome, un radical cyano, méthoxy ou éthoxy, phényle, phényloxy ou phénylthio dont chacun est éventuellement substitué par du fluor, du chlore, du brome, un radical cyano, méthyle, méthoxy, trifluorométhyle et/ou trifluorométhoxy,
R⁵ représente l'hydrogène, le fluor, le chlore, le brome ou un groupe méthyle, éthyle, n-propyle ou isopropyle dont chacun est éventuellement substitué par du fluor et/ou du chlore,
R⁶ est un groupe méthyle, éthyle, n-propyle ou isopropyle éventuellement substitué par du fluor et/ou du chlore,
R⁷ est l'hydrogène, un groupe amino ou bien un groupe méthyle, éthyle, n-propyle, isopropyle, n-butyle, isobutyle, sec.-butyle, méthoxy, éthoxy, n-propoxy, isopropoxy, n-butoxy, isobutoxy, sec.-butoxy, propényle, butényle, propynyle ou butynyle dont chacun est éventuellement substitué par du fluor et/ou du chlore.

4. Procédé de production d'aminophényluraciles substitués suivant l'une des revendications 1 à 3, caractérisé en ce qu'on fait réagir des sulfonylaminophényluraciles de formule générale (II) 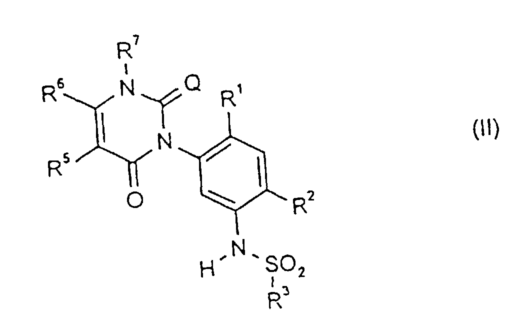 dans laquelle :
Q, R¹, R², R³, R⁵, R⁶ et R⁷ ont les définitions données dans l'une des revendications 1 à 3,
avec des dérivés d'acides de formule générale (III)
R⁴-CO-X (III)
dans laquelle :
R⁴ a la définition indiquée dans l'une des revendications 1 à 3 et
X est un halogène ou le groupement -O-CO-R⁴, éventuellement en présence d'un auxiliaire de réaction et le cas échéant en présence d'un diluant.

5. Procédé pour combattre des plantes indésirables, caractérisé en ce qu'on fait agir des aminophényluraciles substitués suivant l'une des revendications 1 à 3 sur des plantes et/ou sur leur milieu.

6. Utilisation d'aminophényluraciles substitués suivant l'une des revendications 1 à 3 pour combattre des plantes indésirables et des insectes indésirables.

7. Procédé de préparation de compositions herbicides et insecticides, caractérisé en ce qu'on mélange des aminophényluraciles substitués suivant l'une des revendications 1 à 3 avec des diluants et/ou des agents tensioactifs.

8. Compositions herbicides et compositions insecticides, caractérisées par une teneur en au moins un aminophényluracile substitué suivant l'une des revendications 1 à 3.

9. Procédé pour combattre des insectes indésirables, caractérisé en ce qu'on fait agir des aminophényluraciles substitués suivant l'une des revendications 1 à 3 sur les insectes et/ou leur milieu.
